# EUROPÄISCHE PATENTSCHRIFT

(11) **EP 1 220 824 B1**
(45) Veröffentlichungstag und Bekanntmachung des Hinweises auf die Patenterteilung: **11.02.2004**
(21) Anmeldenummer: 00966145.5
(22) Anmeldetag: 10.10.2000
(51) Int. Cl.: C07C 45/74, C07C 45/62, C07C 45/73, C07C 47/232, C07C 47/228

(54) **KONTINUIERLICHES VERFAHREN ZUR HERSTELLUNG VON ZIMTALDEHYD- UND DIHYDROZIMTALDEHYDDERIVATEN**
CONTINUOUS METHOD FOR PRODUCTION OF CINNAMALDEHYDE AND DIHYDROCINNAMALDEHYDE DERIVATIVES
PROCEDE DE PRODUCTION EN CONTINU DE DERIVES D'ALDEHYDE CINNAMIQUE ET D'ALDEHYDE DIHYDROCINNAMIQUE

(30) Priorität: 14.10.1999 DE 19949672
(43) Veröffentlichungstag der Anmeldung: 10.07.2002
(73) Patentinhaber: BASF AKTIENGESELLSCHAFT, 67056 Ludwigshafen (DE)
(72) Erfinder: THERRE, Jörg, 67551 Worms (DE); KRAMER, Andreas, 67098 Bad Dürkheim (DE); WEIPER-IDELMANN, Andreas, 67165 Waldsee (DE); SCHULIK, Michael, 67071 Ludwigshafen (DE); BENFER, Regina, 67122 Altrip (DE); SCHOSSIG, Jürgen, 67136 Fussgönheim (DE); HAAKE, Mathias, 68161 Mannheim (DE); GÖBBEL, Hans-Georg, 68161 Mannheim (DE)
(86) Internationale Anmeldenummer: PCT/EP2000/009918
(87) Internationale Veröffentlichungsnummer: WO 2001/027061

(56) Entgegenhaltungen:
- EP-A- 0 798 039
- FR-A- 2 153 673
- GB-A- 798 901
- US-A- 1 844 013
- US-A- 2 529 186
- US-A- 3 935 274
- US-A- 5 811 588

## Beschreibung

Die vorliegende Erfindung betrifft ein kontinuierliches Verfahren zur Herstellung von Zimtaldehyd oder Zimtaldehydderivaten durch kontinuierliche Umsetzung von Benzaldehydderivaten mit Alkanalen in Gegenwart von Basen und gegebenenfalls anschließender kontinuierlicher Hydrierung in einem Umlaufreaktor in Gegenwart eines Suspensionskatalysators und Wasserstoff zu Dihydrozimtaldehydderivaten.

Zimtaldehydderivate, wie beispielsweise 2-Pentyl-3-phenyl-2-propenal oder das 2-Hexyl-3-phenyl-2-propenal, der Zimtaldehyd selbst, oder die korrespondierenden Dihydroverbindungen beispielsweise Cyclamenaldehyd (2-Methyl-3-(p-isopropyl-phenyl)propanal) oder Lysmeral (2-Methyl-3-(p-tert-butyl-phenyl)propanal) finden als Zwischenprodukte für Riechstoffe, bzw. als Riechstoffe selbst Anwendung und werden darüber hinaus als Ausgangsstoffe für die Synthese von Wirkstoffen im Bereich Pharma und Pflanzenschutz eingesetzt (vgl. GB 1 086 447).

Es ist bekannt, daß man Zimtaldehydderivate durch Umsetzung von Benzaldehydderivaten mit Alkanalen in Gegenwart basischer Katalysatoren, also durch die sogenannte Aldolkondensation, herstellen kann (vgl. Houben-Weyl, "Methoden der organischen Chemie" Band 7/1, S. 76 ff (1954) sowie US 2 976 321. Nach dem bekannten Stand der Technik werden die Reaktionspartner in diskontinuierlichen oder halbkontinuierlichen Verfahren umgesetzt.

So wird in US 2 529 186 von 1947 ein halbkontinuierliches Verfahren zur Herstellung von Zimtaldehyd durch Umsetzung von Benzaldehyd mit Ethanal beschrieben, bei dem der Benzaldehyd in Gegenwart von beispielsweise wäßrigem Alkalihydroxid vorgelegt wird und hierzu das Ethanal langsam in geringem Überschuß zugegeben wird. Das Alkalihydroxid soll hierbei in einer Menge von 2,5 bis 6,5 Gew.-Teilen pro Gew.-Teil Aldehyd angewandt werden. Nachweislich der Beispiele werden für dieses Verfahrens nur Ausbeuten von 75 bis 85 % erhalten. Nachteilig an diesem Verfahren sind sowohl die für eine Anwendung im industriellen Maßstab unbefriedigenden Ausbeuten als auch die benötigte große Menge an Alkalihydroxid, die neben den dafür notwendigen Kosten, eine starke Belastung des Abwassers bedeutet, sowie die relativ lange Reaktionszeit, welche entsprechend große Reaktionsgefäße notwendig macht.

In D.P. B 9977 von 1950 wird ein halbkontinuierliches Verfahren zur Herstellung von Zimtaldehyd durch die Kondensation von Benzaldehyd und Acetaldehyd mittels Alkali in wäßrigem Medium beschrieben. Der Benzaldehyd soll dabei im Überschuß verwendet werden und der Acetaldehyd nur allmählich zugeben werden. Zusätzlich sollen pro Gew.-Teil Benzaldehyd etwa 2 bis 3 Gew.-Teile Wasser angewendet werden. Insbesondere wegen der eingesetzten großen Menge an Wasser, die gereinigt und entsorgt werden muß, ist das Verfahren nicht wirtschaftlich.

In EP 392 579 wird ein Verfahren zur Herstellung von α-substituierten Zimtaldehyden durch Umsetzung von Benzaldehyd mit Alkanalen beschrieben. Als Katalysator wird Alkalihydroxid und als Lösungsmittel Glykol verwendet. Als weitere Hilfsstoffe werden unpolare Kohlenwasserstoffe, wie Hexan verwendet. Es wird hierin ausgeführt (vgl. Seite 2, Zeilen 10-16 und 38-39), daß wegen der Bildung von schlecht abtrennbaren Nebenkomponenten die Verwendung von kontinuierlich durchflossenen Reaktoren ungünstig sei. Dieses Vorurteil wurde durch unseren Arbeiten widerlegt. Es wurde nämlich überraschenderweise gefunden, daß bei Hintereinanderschaltung von mehreren Reaktoren und Einspeisung des Alkanals in mehr als einen der Reaktoren auf relativ einfache Weise ein nebenproduktarmes Zimtaldehydderivat in recht guten Ausbeuten hergestellt werden kann.

Neben einer halbkontinuierlichen Fahrweise wird in EP 392 579 die Durchführung des Verfahrens in einem kontinuierlich durchflossenen Rührkessel mit einer Verweilzeit von 9,5 Stunden beschrieben. Eine mehrstufige Reaktorkaskade wird nicht genannt. Nachteilig bei dem beschriebenen Verfahren ist die erforderliche lange Verweilzeit und die sich daraus ergebenden großen und teuren Reaktionsgefäße. Da die Umsetzung in einem System aus zwei flüssigen, nicht miteinander mischbaren Phasen erfolgt, ist der Scale-up von Laborexperimenten hin zu einer Großanlage zudem sehr schwierig. Weiterhin ist die Regelung des kontinuierlichen durchflossenen Rührkessels und das genaue Einhalten des richtigen Phasenverhältnisses recht schwierig (Steuerung durch Computer erforderlich).

Ein weiterer Nachteil ist die komplizierte Aufarbeitung der Reaktionsmischung, bei der die Glykol enthaltende Reaktionsmischung mehrfach mit Hexan extrahiert werden muß. Wegen der beschriebenen Nachteile ist das Verfahren nicht wirtschaftlich durchführbar.

Um die Nachteile des Verfahrens gemäß EP 392 579 zu überwinden, wird in der europäischen Patentschrift EP 771 780 ein Verfahren zur Herstellung von α-Alkyl-Zimtaldehyd durch Umsetzung von Benzaldehyd mit Alkanal mit Pyrrolidin als basischem Katalysator vorgeschlagen. Als zusätzliche Co-Katalysatoren werden hierin Säuren, wie Schwefelsäure oder Salzsäure, empfohlen. Zur Aufarbeitung soll das rohe Reaktionsprodukt zuerst mit wäßriger Natronlauge gewaschen und dann mit Säure neutralisiert werden.

Nachteilig bei diesem Verfahren ist die Verwendung eines teueren Katalysators, der in großen Mengen zugegeben wird und nicht zurückgeführt werden kann. Dies bedeutet erhöhte Kosten für Einsatzstoffe und Entsorgung. Ein weiterer Nachteil ist die aufwendige, komplizierte Aufarbeitung, bei der das Reaktionsprodukt mit großen Mengen an Lauge gewaschen werden muß. Dieses Vorgehen verursacht wiederum hohe Einsatzstoffkosten und Entsorgungskosten. Da als Co-Katalysatoren Säuren verwendet werden, müssen die Apparaturen aus korrosionsfesten Werkstoffen gefertigt werden. Wegen der oben genannten Nachteile ist das Verfahren unwirtschaftlich.

Bei den bekannten Verfahren zur Herstellung von Zimtaldehydderivaten der Formel II handelt es sich um diskontinuierliche oder halbkontinuierlich Verfahren, welche die aus dieser Fahrweise herrührenden Nachteile aufweisen: lange Reaktionszeiten, große Reaktionsapparate und absatzweiser Betrieb, was beim Einsatz in industriellem Maßstab einen erhöhten Aufwand an Personal und Wartung bedingt. Bei den Verfahren, in denen die Möglichkeit einer kontinuierlichen Verfahrensführung erwähnt wird, fehlen jegliche Angaben zur Reaktionsführung, oder es werden nur einzelne kontinuierlich durchflossene Rührkessel erwähnt, die aus den oben genannten Gründen nicht für eine wirtschaftliche Herstellung von Zimtaldehydderivaten in industriellem Maßstab geeignet sind.

Ebenfalls in der Literatur bereits umfangreich dokumentiert ist die Hydrierung von Zimtaldehyd und dessen Derivaten. Einen Überblick hierzu erhält man durch das Werk Houben-Weyl, Methoden der organischen Chemie, 4. Auflage, 19979, Band 4/1c, Seite 161 f. und gleiches Werk Band 7/1 Seite 388 ff.

Aus US 3,280,192 ist bekannt, daß Zimtaldehyderivate wie Dehydrolysmeral (2-Methy-3-(p-tert.-butyl-phenyl)propenal) durch Umsetzung an palladiumhaltigen Katalysatoren in Gegenwart von Wasserstoff zu den Dihydroverbindungen transformiert werden können. Als besonders vorteilhaft für die Selektivität der Hydrierung geben die Autoren den Zusatz einer wäßrigen Phase an, die nicht mit dem Einsatzstoff mischbar ist und einen pH-Wert zwischen 8 und 13 besitzt. In diesem diskontinuierlichen Verfahren werden gute Ausbeuten und Selektivitäten erreicht.

Eine Verbesserung der Raum-Zeit-Ausbeute wird in DE 26 136 45 erreicht, wenn die Hydrierung im Vergleich zur obigen US-Anmeldung bei höheren Temperaturen von 100 bis 160°C unter mehrmaligem Austausch der Wasserstoffatmosphere durchgeführt wird.

In JP 72 50096 wird ebenfalls in einem Batch-Prozeß zur Hydrierung von Zimtaldehydderivaten an Palladium-Katalysatoren der Zusatz von basischen Verbindungen wie K₂CO₃ empfohlen. Wobei das Edukt ohne Lösungsmittel in einer hohen Reinheit > 97 % eingesetzt wird.

Auch in EP 058 326 wird ein Batch-Verfahren zur Umsetzung eines vorher in situ erzeugten Zimtaldehydderivates an Palladium-Katalysatoren in Gegenwart von Aminen beschrieben.

Eine kontinuierliche Reaktionsführung zur Hydrierung wird in US 3,520,934 beschrieben, die den Zusatz von Kaliumacetat zu Palladium auf Al₂O₃ als Katalysator als besonders vorteilhaft beschreibt.

In US 3,520,935 ist für die kontinuierliche Reaktionsführung die Verwendung eines besonderen lithiumhaltigen Katalysators erforderlich, um gute Raum-Zeit-Ausbeuten bei hohen Umsätzen und Selektivitäten zu erreichen. Angaben zur Standzeit des Katalysators, der in Form von Strängen oder Kugeln in den Reaktor eingesetzt werden muß, werden nicht gemacht.

Insgesamt erfordern die im Stand der Technik aufgeführten Beispiele zur Hydrierung von Zimtaldehydderivaten entweder eine aufwendige diskontinuierliche Reaktionsführung, bei langen Reaktionszeiten und großen Reaktionsapparaten, oder sie bedürfen für eine kontinuierliche Reaktionsführung am Festbett den Einsatz speziell präparierter Katalysatoren, die bei einem Verlust an Aktivität nur in aufwendiger Weise ausgetauscht werden können, was in der Regel ein Abstellen der Anlage zur Folge hat.

Ferner gehen alle im Stand der Technik beschriebenen Verfahren von isoliertem und gereinigtem Produkt aus. Ein kontinuierliches Verfahren, daß die kontinuierliche Herstellung der Zimtaldehydderivate ausgehend von den entsprechenden Benzaldehyden und Alkanalen mit einer anschließenden kontinuierlichen Hydrierung zu Dihydrozimtaldehydderivaten kombiniert, wird im Stand der Technik nicht beschrieben.

Der vorliegenden Erfindung lag daher sowohl die Aufgabe zugrunde ein kontinuierliches Verfahren zur Herstellung von Zimtaldehyd bzw. Zimtaldehydderivaten ausgehend von den entsprechenden Benzaldehyden und Alkanalen zu entwickeln, bei dem die oben genannten Nachteile der bekannten Verfahren vermieden werden können, als auch ein kontinuierliches Verfahren zur Herstellung von Dihydrozimtaldehydderivaten.

Die Aufgabe wurde erfindungsgemäß gelöst, durch ein Verfahren zur
(a) kontinuierlichen Herstellung von Zimtaldehydderivaten der allgemeinen Formel 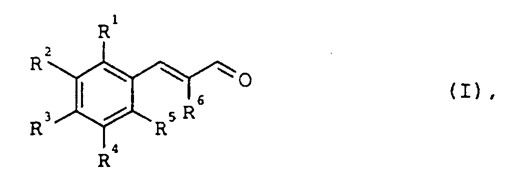 in der R¹, R², R³, R⁴, R⁵ und R⁶ unabhängig voneinander Wasserstoff, C₁₋ bis C₉-Alkylgruppen oder C₁- bis C₉-Alkoxygruppen bedeuten können, vorzugsweise von Zimtaldehydderivaten der allgemeinen Formel I, in der R¹, R², R³ und R⁶ unabhängig voneinander Wasserstoff oder C₁- bis C₉-Alkylgruppen und R⁴ und R⁵ Wasserstoff bedeuten,
   durch Umsetzung von Benzaldehydderivaten der allgemeinen Formel II 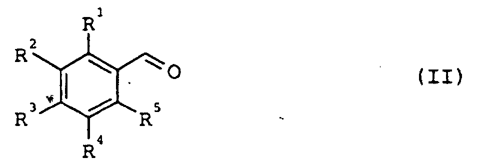 mit Alkanalen der allgemeinen Formel III 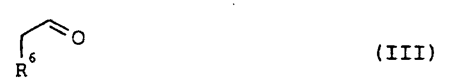 in Gegenwart von Basen, das dadurch gekennzeichnet ist, daß die Reaktanten kontinuierlich in einer Anlage bestehend aus mehreren Reaktoren in Kaskadenschaltung zur Reaktion gebracht werden und daß das Alkanal dabei in mehr als einen der Reaktoren der Anlage eingebracht wird und
(b) gegebenenfalls einer direkt anschließenden kontinuierlicher Hydrierung in einem Umlaufreaktor in Gegenwart eines Suspensionskatalysators und Wasserstoff zu Dihydrozimtaldehydderivaten der allgemeinen Formel IV, 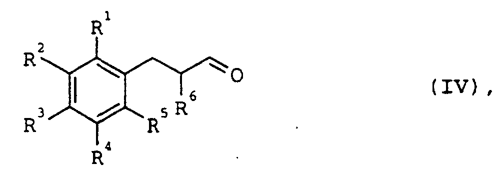
wobei R¹ bis R⁶ die oben angegebene Bedeutung haben.

Gegenstand der Erfindung ist sowohl die kontinuierliche Herstellung des Zimtaldehydderivates der allgemeinen Formel I als auch die Herstellung des Dihydrozimtaldehydderivates der allgemeinen Formel IV durch Kombination der Verfahrensschritte (a) und (b).

Bevorzugt findet im Anschluß an die Herstellung der Zimtaldehydderivate der allgemeinen Formel I eine Hydrierung zu den entsprechenden Dihydrozimtaldehydderivaten der allgemeinen Formel IV statt.

Findet im Anschluß an Stufe (a) eine Hydrierung gemäß (b) statt, zeichnet sich das Verfahren gegenüber dem Stand der Technik zum einen durch die kontinuierliche Reaktionsführung beider Prozesse aus und zum anderen dadurch, daß für die Hydrierung eine Aufreinigung oder Isolierung des Eduktes, welches in der vorgeschalteten Aldolkondensation zwischen dem Benzaldehydderivat der allgemeinen Formel II und dem Alkanal der allgemeinen Formel III hergestellt wird, nicht erforderlich ist.

Der unter (a) erhaltene Reaktionsaustrag wird nach Einstellen eines pH-Wertes zwischen 7 und 13, bevorzugt zwischen 8 und 9 ohne Aufarbeitung der Hydrierung zugeführt.

Zur Einstellung des pH-Wertes eignen sich Brönstedt-Säuren, bevorzugt organische Säuren wie Ameisensäure, Propionsäure, Citronensäure, Phthalsäure, besonders bevorzugt Essigsäure.

Als Benzaldehydderivate der allgemeinen Formel II werden erfindungsgemäß beispielsweise Benzaldehyd selbst, m-Isopropyl-benzaldehyd, p-Isopropyl-benzaldehyd, p-tert.-Butyl-benzaldehyd, m-tert.-Butyl-benzaldehyd, m-Methyl-benzaldehyd, p-Methyl-benzaldehyd, o-Methyl-benzaldehyd, m-Anisaldehyd oder p-Anisaldehyd, oder aber Gemische von 2 oder mehreren dieser Benzaldehydderivate verwendet.

Als für die erfindungsgemäße Umsetzung geeignete Alkanale der allgemeinen Formel IV seien insbesondere der Acetaldehyd, Propanal, n- und iso-Butanal, n- und iso-Pentanal, n- und iso-Hexanal genannt.

Bevorzugte Dihydrozimtaldehydderivate der Formel IV sind Cyclamenaldeyd oder Lysmeral. Insbesonder bevorzugt wird mit dem erfindungsgemäßen Verfahren (Stufe (a) und Stufe (b)) Lysmeral hergestellt.

Als Katalysator zur Herstellung der Zimtaldehydderivate der allgemeinen Formel I (Stufe (a) für das erfindungsgemäße Verfahren werden basische Verbindungen, wie die Alkalihydroxide NaOH, KOH und LiOH, Erdalkalihydroxide sowie deren Gemische oder aber Verbindungen der Alkali- und Erdalkalimetalle, die in Gegenwart von Wasser die korrespondierenden Hydroxide freisetzen können, sowie Ammoniak, Amine oder Alkoholate, wie beispielsweise Natriummethylat oder Kalium-tert.-butylat, verwendet. Mit besonderem Vorteil arbeitet man mit wäßrigen Lösungen der Alkalimetalloxide und -hydroxide, insbesondere Lösungen von Natriumoxid oder -hydroxid, wobei die Konzentration des Oxids bzw. des Hydroxids zwischen 0,5 und 50 Gew.-%, vorzugsweise zwischen 10 und 50 Gew.-% beträgt. Die Base wird im allgemeinen vollständig in den ersten Reaktor eingebracht, kann aber auch auf alle Reaktoren verteilt werden. Die Menge der benötigten Base ist sehr gering, was einen besonderen Vorteil des erfindungsgemäßen Verfahrens darstellt. So soll die Konzentration der Base in der Reaktionsmischung nur zwischen 0,01 Gew.-% und 20 Gew.-%, vorzugsweise zwischen 0,1 Gew.-% und 5 Gew.-% betragen.

Die Stufe (a) des erfindungsgemäßen Verfahrens wird in den Beispielen 1 bis 4 unter Verwendung einer aus 3 Reaktoren bestehenden Kaskade verdeutlicht.

Die bevorzugte Anlage zur erfindungsgemäßen kontinuierlichen Herstellung der Zimtaldehydderivate der Formel I besteht prinzipiell aus mehreren hintereinander geschalteten Reaktoren in Kaskadenschaltung. Die Zahl der verwendeten Reaktoren liegt im allgemeinen zwischen 2 und 20, vorzugsweise zwischen 2 und 10 und insbesondere zwischen 2 und 5 Reaktoren. Die Reaktoren werden in Kaskadenschaltung betrieben, was bedeutet, daß der Ablauf des einen Reaktors jeweils in den nächsten Reaktor geleitet wird. Am Ausgang des letzten Reaktors wird dann das gebildete Reaktionsgemisch entnommen.

In einer weniger bevorzugten Ausgestaltung des Verfahrens kann auch Produktgemisch aus einem oder mehreren der hinteren Reaktoren entnommen und in einen oder mehrere der vorderen Reaktoren zurückgeführt werden.

Als Reaktoren sind nicht rückvermischte Reaktoren, wie Rohrreaktoren oder mit Einbauten versehene Verweilzeitbehälter, vorzugsweise aber rückvermischte Reaktoren, wie Rührkessel, Schlaufenreaktoren, Strahlschlaufenreaktoren oder Strahldüsenreaktoren geeignet. Es können aber auch Kombinationen aus aufeinander folgenden rückvermischten Reaktoren und nicht rückvermischten Reaktoren verwendet werden.

Gegebenenfalls können auch mehrere Reaktoren in einer mehrstufigen Apparatur zusammengefaßt werden. Solche Reaktoren sind zum Beispiel Schlaufenreaktoren mit eingebauten Siebböden, kaskadierte Behälter, Rohrreaktoren mit Zwischeneinspeisung oder Rührkolonnen. Es empfiehlt sich die einzelnen Reaktoren alle oder teilweise mit Wärmetauschern auszustatten. In den Reaktoren muß für eine gute Durchmischung der Reaktanten gesorgt werden, was beispielsweise durch Rühren oder Umpumpen, gegebenenfalls in Kombination mit dem Behandeln mit statischen Mischern oder Mischdüsen erfolgen kann.

Das Volumen der Reaktoren ist so zu bemessen, daß die mittlere Verweilzeit der Reaktionsmischung in den Reaktoren zwischen 5 Minuten und 8 Stunden, insbesondere zwischen 10 Minuten und 5 Stunden beträgt. Mit besonderem Vorteil arbeitet man mit Reaktoren, die etwa das gleiche Volumen aufweisen, prinzipiell können aber auch Reaktoren mit unterschiedlichen Volumina eingesetzt werden.

Die Temperatur in den Reaktoren liegt im allgemeinen zwischen 20 und 130°C, insbesondere zwischen 30 und 100°C.

Der Druck in den Reaktoren ist nicht kritisch, er sollte aber so hoch sein, daß der Inhalt der Reaktoren nahezu flüssig bleibt. Im allgemeinen sind dazu Drücke von 1 bar bis 40 bar erforderlich, mit Vorteil liegt der Druck zwischen 1 und 6 bar.

Der Benzaldehyd bzw. das Benzaldehydderivat der allgemeinen Formel II wird vorzugsweise in den ersten Reaktor der Anlage mit Kaskadenschaltung eingebracht. In einigen Fällen kann es aber auch erforderlich sein, Teilmengen des Benzaldehyd(derivate)s in die übrigen Reaktoren der Anlage mit Kaskadenschaltung zuzugeben.

In einer besonders bevorzugten Ausführung des erfindungsgemäßen Verfahrens wird der Benzaldehyd bzw. das Benzaldehydderivat vor dem Einbringen in die Reaktoren mit dem Teil des Alkanals vorgemischt, das in den selben Reaktor zugegeben wird. Zu diesem Vormischen können die üblichen Mischapparate, wie statische Mischer, Rührkessel, Mischdüsen oder ähnliches verwendet werden. Das Vorvermischen der beiden Reaktanten führt zu einer Steigerung der Selektivität. Diese Selektivitätssteigerung ist besonders überraschend, da in der Patentschrift US 2 529 186 ausgeführt wird, daß ein Vorvermischen der Reaktanten eine Verringerung der Selektivität bewirken würde.

Das Alkanal soll in mehr als einen Reaktor der Reaktionsanlage zugegeben werden. Besonders vorteilhaft ist es, wenn in jeden Reaktor der Anlage in Kaskadenschaltung ein Teil des Alkanals zugegeben wird. Weiterhin wurde aber auch gefunden, daß eine gleichmäßige Verteilung des Alkanals auf alle Reaktoren ungünstig für eine hohe Selektivität ist. Es hat sich daher als vorteilhaft erwiesen daß man in den ersten Reaktor zwischen etwa 20 % und 70 % der insgesamt zuzugebenden Menge an Alkanal, insbesondere zwischen 40 und 60 % des Alkanals einbringt und die restliche Menge, d.h. etwa 80 bis 30 % an Alkanal in den oder die weiteren Reaktoren zugibt. Weiterhin ist es besonders vorteilhaft, wenn man wenigstens einen Teil des Alkanals mit einem Teil oder der Gesamtmenge des Benzaldehydderivats vorvermischt in den Reaktor einbringt.

Das Alkanal wird im allgemeinen in flüssiger Form eingesetzt. Prinzipiell ist aber auch eine gasförmige Zugabe möglich.

In einer besonders bevorzugten Ausführung des Verfahrens wird die Menge des Alkanals so gewählt, daß das molare Verhältnis aus Benzaldehyd(derivat) zu Alkanal in allen Reaktoren zwischen 5 und 100 mol/mol, vorzugsweise zwischen 10 und 60 mol/mol liegt. Dieses molare Verhältnis kann durch die Analyse der Reaktionsmischung mittels bekannter analytischer Methoden, beispielsweise durch Gaschromatographie leicht bestimmt werden. Im Allgemeinen genügt eine einzige Analyse pro Tag, in besonderen Fällen kann aber auch eine Online-Analytik erforderlich sein.

In Abhängigkeit von der Temperatur, der Verweilzeit und der Menge an Base beträgt der Umsatz des Benzaldehyd(derivate)s zwischen 20 % und 95 %, vorzugsweise zwischen 30 % und 80 %. Ein besonderer Vorteil des erfindungsgemäßen Verfahren besteht darin, daß der Umsatz des Alkanals derart hoch ist, daß in der Regel auf eine Rückgewinnung des nicht umgesetzten Alkanals verzichtet werden kann.

Zur Steigerung der Selektivität können zu dem Reaktionsgemisch Lösungsmittel zugegeben werden. Als Lösungsmittel sind Alkohole, wie Methanol, Ethanol, Propanol, Isopropanol, Butanol oder Ethylenglykole, oder auch Ether, wie Diethylether oder cyclische Ether, wie Dioxan, geeignet. Mit besonderem Vorteil verwendet man als Lösungsmittel Methanol. Das Lösungsmittel kann unvermischt oder aber mit einem oder mehreren der Reaktanten oder der Base vermischt in die einzelnen Reaktoren eingebracht werden. Bevorzugt wird das Lösungsmittel aber vollständig in den ersten Reaktor zugegeben.

Der Austrag der Reaktionsanlage kann mittels der üblichen Trennoperationen, wie Kristallisation, Phasentrennung oder fraktionierende Destillation leicht aufgearbeitet und das Zimtaldehydderivat so auf einfache Weise isoliert werden.
Im allgemeinen ist es von Vorteil, vor der Aufarbeitung des Reaktionsgemisches den basischen Katalysator durch Zugabe von anorganischen oder organischen Säuren, zumindest teilweise zu neutralisieren.

Mit dem erfindungsgemäßen Verfahren (Stufe (a)) können Zimtaldehyd und allgemein Zimtaldehydderivate der Formel I in hoher Reinheit, sehr guter Qualität auf einfache Art und Weise hergestellt werden. Für das erfindungsgemäße Verfahren sind nur wenige, einfache, kleine und kostengünstige Apparate erforderlich. Mit Hilfe des erfindungsgemäßen Verfahrens kann man die Zimtaldehydderivate der Formel I in hohen Selektivitäten, bezogen sowohl auf das Alkanal als auch auf das Benzaldehydderivat, herstellen.

Findet im Anschluß an Stufe (a) eine Hydrierung gemäß Stufe (b) zum Dihydrozimtaldehydderivat statt, so wird sie in einem Reaktor gemäß US 5,939,589 durchgeführt, in dem die Flüssig- und die Gasphase durch eine Vorrichtung mit Öffnungen oder Kanälen mit einem hydraulischen Durchmesser von 0,5 bis 20 mm, vorzugsweise 1 bis 10 mm, besonders bevorzugt 1 bis 3 mm durchgeleitet wird. Der hydraulische Durchmesser ist als Quotient des 4-fachen Querschnitts der Öffnung und deren Umfang definiert.

Die Vorrichtung mit Öffnungen oder Kanälen zur Durchleitung des Reaktionsmediums kann aus einer Schüttung, einem Gestrick, einer offenzelligen Schaumstruktur, vorzugsweise aus Kunststoff (z.B. Polyurethan oder Melaminharze) oder Keramik, oder einem Packungselement, wie es grundsätzlich, d.h. seiner geometrischen Form nach, bereits aus der Destillations- und Extraktionstechnik bekannt ist, bestehen.

Derartige Packungselemente, die den Vorteil eines geringen Druckverlustes bieten, sind z.B. Drahtgewebepackungen. Für Zwecke der vorliegenden Erfindung haben die Packungen jedoch grundsätzlich einen wesentlich, regelmäßig um den Faktor 2 bis 10 kleineren hydraulischen Durchmesser als vergleichbare Einbauten im Bereich der Destillations- und Extraktionstechnik.

Statt Gewebepackungen können auch Packungen aus anderen gewebten, gewelkten oder gefilzten flüssigkeitsdurchlässigen Materialien verwendet werden. Weitere geeignete Packungen sind Blechpackungen. Vorteilhaft sind auch Packungen aus Streckmetall, wie zum Beispiel Packungen des Typs Montz BSH. Die Öffnungen, z.B. Perforationen müssen entsprechend klein gehalten werden. Entscheidend für die Eignung einer Packung im Rahmen der vorliegenden Erfindung ist nicht deren Geometrie, sondern die für die Stromführung entstehenden Öffnungsgrößen bzw. Kanalbreiten in der Packung.

Bei der Hydrierung können zur Suspension handelsübliche Katalysatorteilchen mit einer mittleren Korngröße von 0,0001 bis 2 mm, bevorzugt von 0,001 bis 0,1 mm, besonders bevorzugt von 0,005 bis 0,05 mm verwendet werden.

Die Hydrierung wird nach dem erfindungsgemäßen Verfahren in einem Reaktor mit einer der oben beschriebenen Einbauten in Gegenwart von Wasserstoff bei einem Druck zwischen 1 und 100 bar, bevorzugt 1 und 30 bar, besonders bevorzugt 1 und 15 bar durchgeführt.
Die Reaktionstemperaturen liegen zwischen 10 und 160°C, bevorzugt zwischen 20 und 80°C, besonders bevorzugt zwischen 40 und 75°C.

Als Einbauten im Hydrierreaktor werden Gewebepackungen oder Blechpackungen verwendet. Reaktionsgemisch, Katalysator und Wasserstoff werden mit hoher Geschwindigkeit im Kreis durch den Reaktor gepumpt. Die Querschnittsflächenbelastung von Gas- und Flüssigphase liegen hierbei über 50 m³/m²h, bevorzugt im Bereich von 50 bis 300 m³/m²h, besonders bevorzugt im Bereich von 100 bis 250 m³/m²h. Die Gasphase wird mittels einer Injektordüse inniglich mit der Flüssigphase durchmischt.

Für die Hydrierung wird ein kommerziell verfügbarer Suspensionskatalysator verwendet, der bevorzugt als Aktivkomponente mindestens Palladium enthält. Neben Palladium kann der Katalysator auch weitere Aktivkomponenten wie beispielsweise Zink, Cadmium, Platin, Silber oder ein Metall der Seltenen Erden enthalten.
Der Katalysator kann in metallischer und/oder oxidischer Form unter anderem auf Trägermaterialien eingesetzt werden. Als Trägermaterialien eignen sich beispielsweise SiO₂, TiO₂, ZrO₂, Al₂O₃ oder Kohlenstoff wie Graphite, Ruße oder Aktivkohle, insbesondere bevorzugt wird Aktivkohle. Der Gehalt an Palladium liegt zwischen 0,1 und 10 Gew.-%, bevorzugt zwischen 1 und 7 Gew.-%.

Die erfindungsgemäße Hydrierung zur Herstellung von Dihydrozimtaldehydderivaten zeichnet sich dadurch aus, daß die Flüssigkeit mit einer Leerrohrgeschwindigkeit von etwa 50 bis 300 m³/m²h, vorzugsweise von 250 bis 200 m³/m²h, durch die oben beschriebene Vorrichtung mit Öffnungen und Kanälen geführt wird. Bei gleichzeitiger Anwesenheit der Gasphase beträgt deren Leerrohrgeschwindigkeit vorzugsweise 50 bis 300 m³/m²h, besonders bevorzugt 100 bis 200 m³/m²h.

Die vorliegende Hydrierung kann in verschiedenen kontinuierlich betriebenen Reaktorbauformen, wie Strahldüsenreaktoren, Blasensäulen oder Rohrbündelreaktoren durchgeführt werden. Die oben aufgeführten Einbauten füllen vorzugsweise aber nicht notwendigerweise den gesamten Reaktor aus. Der erfindungsgemäße Reaktor ist vorzugsweise eine vertikal angeordnete Blasensäule, die bei Anwesenheit einer Gasphase bevorzugt im Gleichstrom von unten nach oben durchströmt wird. Ein anderer bevorzugter Reaktor ist ein heiz- und kühlbarer Rohrbündelreaktor, bei dem die erfindungsgemäßen Einbauten in den einzelnen Rohren untergebracht sind. Bei Anwesenheit einer Gasphase wird der Reaktor bevorzugt im Gleichstrom von unten nach oben durchströmt. Das suspendierte Katalysatormaterial kann mit Hilfe gebräuchlicher Techniken eingebracht und wieder abgetrennt werden (Sedimentation, Zentrifugation, Kuchenfiltration, Querstromfiltration).

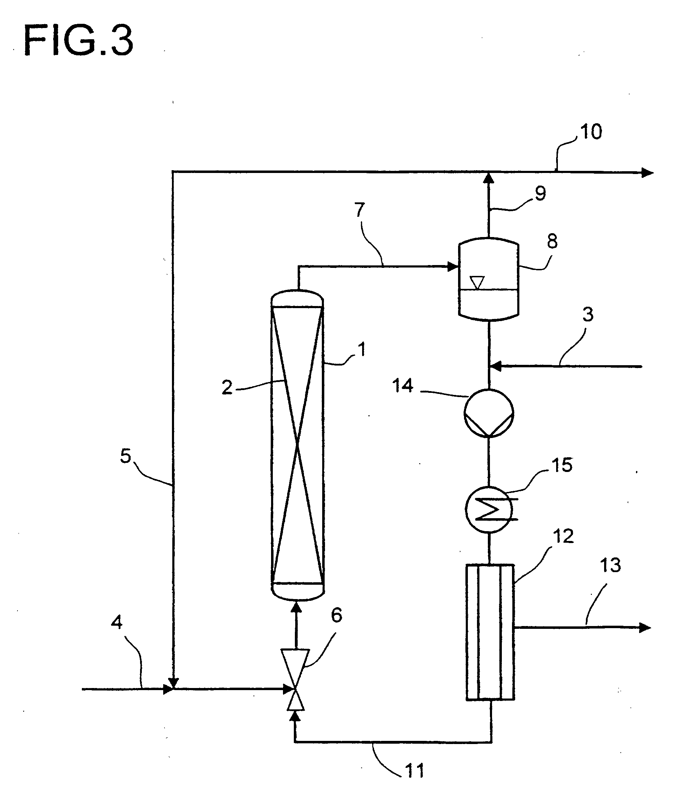

Beispielhaft wird ein Reaktor zur Hydrierung von Dehydrolysmeral zu Lysmeral mit einem Suspensionskatalysator gemäß der vorliegenden Erfindung der Stufe (b) anhand der Figur 3 im Detail beschrieben. Figur 3 zeigt beispielhaft den Versuchsaufbau eines kontinuierlich betriebenen Reaktors (Blasensäule) 1 mit Packung 2, die über die Leitungen 3 mit Flüssigkeit und über die Leitung 4 mit Frischgas gespeist wird. Das Kreisgas 5 wird mittels der Mischdüse 6 mit Frischgas und der durch die Pumpe 14 im Kreis geführten Suspension 11 eingemischt. Der Reaktoraustrag wird über die Leitung 7 in das Abscheidegefäß 8 gefahren, in dem die Gasphase abgeschieden und über Leitung 9 abgeführt wird. Von dieser Gasmenge wird zur Begrenzung der Aufpegelung von gasförmigen Verunreinigungen ein Teilstrom über die Leitung 10 entnommen und die verbleibende Restmenge über die Leitung 5 in den Reaktor geführt. Der suspendierte Katalysator verbleibt im Reaktorsystem, indem er über einen Querstromfilter 12 zurückgehalten und nur katalysatorfreie Flüssigphase über die Leitung 13 austritt und entnommen wird. Über den Wärmetauscher 15 kann die Temperatur im Reaktorsystem gezielt eingestellt werden.

Die folgenden Beispiele sollen die Erfindung näher erläutern, ohne sie jedoch darauf zu beschränken:

### Aldolkondensation (Verfahrensstufe (a))

### Beispiel 1

### (ohne Vormischung)

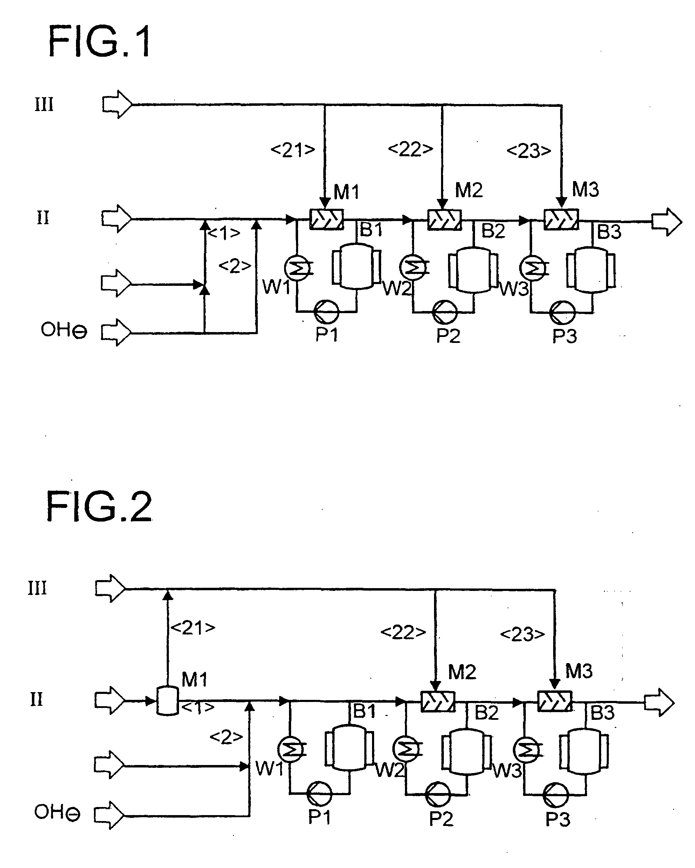

Die Apparatur (vgl. Figur 1) bestand aus drei in Reihe geschalteten Behältern B1, B2 und B3 mit einem Volumen von jeweils 275 ml, die jeder über einen Doppelmantel mit Hilfe eines Thermostaten beheizt und über die Wärmetauscher W1, W2 und W3 gekühlt wurden und deren Inhalte mit den Pumpen P1, P2 und P3 umgewälzt wurden. Das Benzaldehydderivat wurde gemischt mit dem Lösungsmittel und einem Teil der Base in den ersten Behälter zugegeben (Strom <1>). Der zweite Teil der Base wurde in den ersten Behälter gegeben (Strom <2>). Das Alkanal wurde mittels der Mischdüsen M1, M2 und M3 in die entsprechenden Behälter eingemischt (Ströme <21>, <22>, <23>).

In den ersten Behälter wurden als Strom <1> 217 ml/h eine Mischung bestehend aus 54 Gew.-% Methanol, 45,5 Gew.-% p-tert.-Butyl-benzaldehyd und 0,5 % einer 50 gew.-%igen wäßrigen Natronlauge, als Strom <21> 13,1 g/h Propanal und als Strom <2> 6 g/h Natronlauge (50 %ig in Wasser) zudosiert. In den zweiten Behälter wurden als Strom <22> 6,6 g/h Propanal und in den dritten Behälter als Strom <23> 3,3 g/h Propanal zudosiert. Die Temperaturen der getrennt beheizbaren Behälter betrugen zwischen 47°C und 48°C. Die Menge der umgepumpten Flüssigkeit wurde in jedem Behälter auf 600 ml/min festgesetzt. Nach einer Betriebszeit der Kaskade von 8 h hatte der Austrag des dritten Behälters folgende Zusammensetzung: < 0,1 Gew.-% Propanal; 21 Gew.-% p-tert.-Butyl-benzaldehyd 20,5 Gew.-%. 3-(p-tert.-Butyl-phenyl)-2-methyl-2-propenal. Dies entspricht einem Umsatz von 48 % der Menge an p-tert.-Butyl-benzaldehyd, die Selektivität, bezogen auf das Benzaldehydderivat, beträgt 85 %, bezogen auf das Propanal, 62 % der Theorie.

### Beispiel 2

### (ohne Vormischung)

Es wurde die in Beispiel 1 beschriebene Apparatur verwendet.

In den ersten Behälter wurden als Strom <1> 217 ml/h ein Gemisch, bestehend aus 54 Gew.-% Methanol, 45,5 Gew.-% p-tert.-Butylbenzaldehyd und 0,5 Gew.-% einer 50 gew.-%igen wäßrigen Natronlauge, als Strom <21> 13,1 g/h Propanal und als Strom <2> 6 g/h Natronlauge (50 %ig in Wasser) zudosiert. In den zweiten Behälter wurden als Strom <22> 6,6 g/h Propanal und in den dritten Behälter als Strom <23> 3,3 g/h Propanal zudosiert. Die Temperaturen der getrennt beheizbaren Behälter betrugen zwischen 47°C und 48°C. Die Menge der umgepumpten Flüssigkeit wurde in jedem Behälter auf 1200 ml/min eingestellt. Nach einer Betriebszeit der Kaskade von 8 h hatte der Austrag des dritten Behälters folgende Zusammensetzung: < 0,1 Gew.-% Propanal; 19 Gew.-% p-tert.-Butylbenzaldehyd und 23,5 Gew.-%. 3-(p-tert.-Butylphenyl)-2-methyl-2-propenal. Dies entspricht einem Umsatz an p-tert.-Butyl-benzaldehyd von 52 %. Die Selektivität an 3-(p-tert.-Butyl-phenyl)-2-methyl-2-propenal betrug somit 87 % der Theorie, bezogen auf das Benzaldehydderivat, und 70 Gew.-%, bezogen auf das Propanal.

### Beispiel 3

### (mit Vormischen von p-tert.-Butyl-benzaldehyd und Propanal)

Die Apparatur (vgl. Figur 2) bestand aus drei in Reihe geschalteten Behältern B1, B2 und B3 mit einem Volumen von jeweils 275 ml, die jeweils über einen Doppelmantel mit Hilfe eines Thermostaten beheizt und über die Wärmetauscher W1, W2 und W3 gekühlt wurden. Der Inhalt dieser Behälter wurde mit den Pumpen P1, P2 und P3 umgewälzt. Das Benzaldehydderivat und die für den ersten Behälter bestimmte Teilmenge des Alkanals wurden in dem Mischbehälter M1 vorgemischt und dann in den ersten Behälter B1 zu dosiert (Strom <1>). Die für den zweiten und dritten Behälter bestimmte Menge an Alkanal wurde mittels der Mischdüsen M2 und M3 in die entsprechenden Behälter eingemischt (Ströme <22> und <23>). Das Lösungsmittel wurde mit der Base gemischt und die erhaltene Mischung in den ersten Behälter eingebracht (Strom <2>).

In den ersten Behälter wurden als Strom <1> 114 g/h eines Gemisches, bestehend aus 87,7 Gew.-% p-tert.-Butyl-benzaldehyd und 12,3 Gew.-% Propanal und als Strom <2> 129,7 g/h einer Mischung, bestehend aus 4 Gew.-% wäßriger Natronlauge (50 gew.-%ig) und 96 Gew.-% Methanol zudosiert. In den zweiten Behälter wurden als Strom <22> 6,6 g/h an Propanal und in den dritten Behälter wurden als Strom <23> 4,2 g/h an Propanal zudosiert. Die Temperaturen der getrennt beheizbaren Behälter betrugen zwischen 43°C und 51°C. Die Menge der umgepumpten Flüssigkeit wurde in jedem Behälter auf 1200 ml/min eingestellt. Nach einer Betriebszeit der Kaskade von 8 h hatte der Austrag des dritten Behälters folgende Zusammensetzung: < 0,1 Gew.-% Propanal, 16,2 Gew.-% p-tert.-Butyl-benzaldehyd und 28,3 Gew.-% 3-(p-tert.-Butyl-phenyl)-2-methyl-2-propenal. Dies entspricht einem Umsatz an p-tert.-Butyl-benzaldehyd von 60 % der Theorie. Die Selektivität, bezogen auf das Benzaldehydderivat betrug somit 94 % der Theorie und 83 % der Theorie, bezogen auf das Propanal.

### Beispiel 4

### (mit Vormischen von p-tert.-Butyl-benzaldehyd und Propanal, Katalysator Natriummethanolat)

Es wurde die in Beispiel 3 beschriebene und in Figur 2 schematisch dargestellte Apparatur verwendet.

In den ersten Behälter wurden als Strom <1> 114 g/h eines Gemisches, bestehend aus 87,7 Gew.-% p-tert.-Butyl-benzaldehyd und 12,3 Gew.-% Propanal und als Strom <2> 123 g/h eines Gemisches, enthaltend 1 mol Natriummethanolat pro mol Methanol zudosiert. In den zweiten Behälter wurden als Strom <22> 6,6 g/h Propanal und in den dritten Behälter als Strom 203 4,2 g/h Propanal zudosiert. Die Temperaturen der getrennt beheizbaren Behälter betrugen zwischen 43 und 50°C. Die Menge der umgepumpten Flüssigkeit wurde in jedem Behälter auf 1200 ml/min eingestellt. Nach einer Betriebszeit der Kaskade von 8 h hatte der Austrag des dritten Behälters folgende Zusammensetzung: < 0,1 Gew.-% Propanal, 20,4 Gew.-% p-tert.-Butyl-benzaldehyd und 20,3 Gew.-% 3-(p-tert.-Butyl-phenyl)-2-methyl-2-propenal. Dies entspricht einem Umsatz an p-tert.-Butyl-benzaldehyd von 49 %. Die Selektivität an 3-(p-tert.-Butyl-phenyl)-2-methyl-2-propenal, betrug somit 83 % der Theorie, bezogen auf das Benzaldehydderivat und 59 % der Theorie, bezogen auf das Propanal.

### Beispiel 5

### (mit Vormischen von p-tert.-Butyl-benzaldehyd und Propanal, Katalysator Kalilauge)

Es wurde die in Beispiel 3 beschriebene und in Figur 2 schematisch dargestellte Apparatur verwendet.

In den ersten Behälter wurden als Strom <1> 114 g/h eines Gemisches, bestehend aus 87,7 Gew.-% p-tert.-Butyl-benzaldehyd und 12,3 Gew.-% Propanal und als Strom <2> 123 g/h eines Gemisches, bestehend aus 5 Gew.-% wäßriger Kalilauge (25 gew.-%ig) und 95 Gew.-% Methancl zudosiert. In den zweiten Behälter wurden als Strom <22> 6,6 g/h Propanal und in den dritten Behälter als Strom <23> 4,2 g/h Propanal zudosiert. Die Temperaturen der getrennt beheizbaren Behälter betrugen zwischen 48 und 51°C. Die Menge der umgepumpten Flüssigkeit wurde in jedem Behälter auf 1200 ml/min eingestellt. Nach einer Betriebszeit der Kaskade von 24 h hatte der Austrag des dritten Behälters folgende Zusammensetzung: < 0,1 Gew.-% Propanal, 16,4 Gew.-% p-tert.-Butyl-benzaldehyd und 30,8 Gew.-% 3-(p-tert.-Butyl-phenyl)-2-methyl-2-propenal. Dies entspricht einem Umsatz an p-tert.-Butylbenzaldehyd von 58 %. Die Selektivität an 3-(p-tert.-Butylphenyl)-2-methyl-2-propenal, betrug somit 99 % der Theorie, bezogen auf das Benzaldehydderivat und 91 % der Theorie, bezogen auf das Propanal.

### Hydrierung (Verfahrensstufe (b))

Die Reaktion wurde in einer mit einer Gewebepackung bestückte Blasensäule (1000 mm, 27,3 mm Durchmesser) entsprechend der vorliegenden Erfindung zur Hydrierung verwendet. Der Versuchsaufbau entsprach Figur 3. Die Geometrie der Packung entsprach einer handelsüblichen Gewebepackung vom Typ Montz A1 1200. Die volumenbezogene Oberfläche der Packung beträgt 1200 m²/m³, wobei sich diese Angabe nur auf die Oberfläche der Gewebe bezieht. Die Flüssigkeit mit dem suspendierten Katalysator und das Gas wurden mit einer Leerrohrgeschwindigkeit von 200 m³/m²h von unten in den gepackten Reaktor eingebracht.

Die Reaktion erfolgte kontinuierlich unter einem Wasserstoffdruck von 10 bar. Als Katalysator wurde ein herkömmlicher Suspensionskatalysator mit einem Gehalt von 5 % Palladium auf Aktivkohle verwendet, der eine mittlerer Korngröße um 30 µm aufwies.

### Beispiel 6

Als Zulauf für die gepackte Blasensäule wurde der Austrag aus der kontinuierlich geführten Aldolkondensation von p-tert.-Butylbenzaldehyd (TBA) mit Propanal nach Einstellung eines pH-Wertes von 8,6 ohne destillative Aufreinigung verwendet. Eine solche Zulauflösung hat üblicherweise die Zusammensetzung 50 Gew.-% Methanol, 29 Gew.-% Dehydrolysmeral (DHL), 13 Gew.-% TBA, 5 Gew.-% Wasser, 1 Gew.-% Schwersieder etc. Die Temperatur wurde mit Hilfe von Thermostaten auf 60°C eingestellt. Der Umsatz betrug > 96 % bei einer Selektivität von > 93 %. Die Katalysatorbelastung beträgt 5,8 kg_{DHL}/ (kg_{Kat*}h), die Raumzeitausbeute 118 kg_{DHL}/ (m³h).

### Beispiel 7

Als Zulauf für die gepackte Blasensäule wurde ein wie oben beschriebener Zulauf (Beispiel 6) verwendet der zusätzlich im Verhältnis 1 : 2 mit Austrag aus der Hydrierung verdünnt wurde. Eine solche Zulauflösung hat üblicherweise die Zusammensetzung 50 Gew.-% Methanol, 20 Gew.-% Lysmeral, 10 Gew.-% Dehydrolysmeral, 13 Gew.-% TBA, 5 Gew.-% Wasser, 1 Gew.-% Schwersieder etc. Die Temperatur wurde auch in diesem Fall mit Hilfe von Thermostaten auf 60°C eingestellt. Die Zulaufmenge betrug 600 g/h. Unter diesen Bedingungen konnte ein Umsatz von 98,2 % und gleichzeitig eine Selektivität von 88,4 % erreicht werden. Die Raum-Zeit-Ausbeute betrug 103 kg_{DHL}/m³*h, die Katalysatorbelastung 3,7 kg_{DHL}/ (kg_{Kat*}h).

### Beispiel 8

Als Zulauf für die gepackte Blasensäule wurde ein wie oben beschriebener Zulauf (Beispiel 6) verwendet der zusätzlich im Verhältnis 1 : 2 mit Austrag aus der Hydrierung verdünnt wurde. Eine solche Zulauflösung hat üblicherweise die Zusammensetzung 50 Gew.-% Methanol, 20 Gew.-% Lysmeral, 10 Gew.-% Dehydrolysmeral, 13 Gew.-% TBA, 5 Gew.-% Wasser, 1 Gew.-% Schwersieder etc. Die Temperatur wurde auch in diesem Fall mit Hilfe von Thermostaten auf 60°C eingestellt. Die Zulaufmenge betrug 900 g/h. Unter diesen Bedingungen konnte ein Umsatz von 94,5 % und gleichzeitig eine Selektivität von 90 % erreicht werden. Die Raum-Zeit-Ausbeute betrug 253 kg_{DHL}/m³*h, die Katalysatorbelastung 8,5 kg_{DHL}/ (kg_{Kat*}h).

## Patentansprüche

1. Verfahren zur
(a) kontinuierlichen Herstellung von Zimtaldehydderivaten der allgemeinen Formel 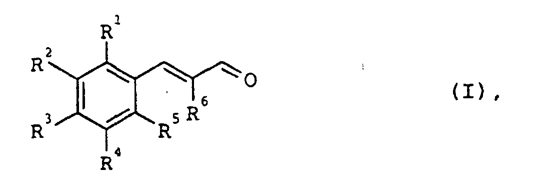 in der R¹, R², R³, R⁴, R⁵ und R⁶ unabhängig voneinander Wasserstoff, C₁₋ bis C₉-Alkylgruppen oder C₁- C₉-Alkoxygruppen bedeuten können, vorzugsweise von Zimtaldehydderivaten der allgemeinen Formel I, in der R¹, R², R³ und R⁶ unabhängig voneinander Wasserstoff oder C₁-C₉-Alkylgruppen und R⁴ und R⁵ Wasserstoff bedeuten,
durch Umsetzung von Benzaldehydderivaten der allgemeinen Formel II 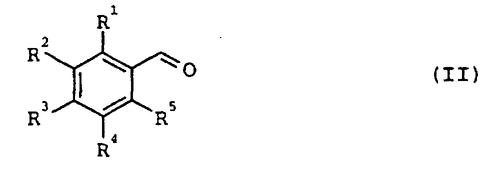 mit Alkanalen der allgemeinen Formel III 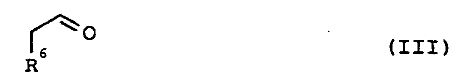 in Gegenwart von Basen, das **dadurch gekennzeichnet ist, daß** die Reaktanten kontinuierlich in einer Anlage bestehend aus mehreren Reaktoren in Kaskadenschaltung zur Reaktion gebracht werden und daß das Alkanal dabei in mehr als einen der Reaktoren der Anlage eingebracht wird und
(b) gegebenenfalls anschließender kontinuierlicher Hydrierung in einem Umlaufreaktor in Gegenwart eines Suspensionskatalysators und Wasserstoff zu Dihydrozimtaldehydderivaten der allgemeinen Formel IV, 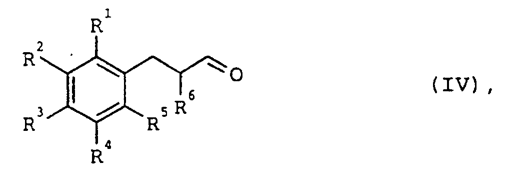
wobei R¹ bis R⁶ die oben angegebene Bedeutung haben.

2. Verfahren gemäß Anspruch 1, **dadurch gekennzeichnet, daß** im Anschluß an Stufe (a) eine Hydrierung gemäß Stufe (b) stattfindet.

3. Verfahren gemäß Anspruch 1 oder 2, **dadurch gekennzeichnet, daß** man als Benzaldehydderivat der Formel II, Benzaldehyd selbst, m-Isopropyl-benzaldehyd, p-Isopropyl-benzaldehyd, p-tert.-Butyl-benzaldehyd, m-tert.-Butyl-benzaldehyd, m-Methyl-benzaldehyd, p-Methyl-benzaldehyd, o-Methyl-benzaldehyd, m-Anisaldehyd oder p-Anisaldehyd, oder aber Gemische von 2 oder mehreren dieser Benzaldehydderivate verwendet.

4. Verfahren gemäß einem der Ansprüche 1 bis 3, **dadurch gekennzeichnet, daß** die Zahl der unter (a) verwendeten Reaktoren in der Anlage zwischen 2 und 20 liegt.

5. Verfahren gemäß einem der Anspürche 1 bis 4, **dadurch gekennzeichnet, daß** die Zahl der unter (a) verwendeten Reaktoren in der Anlage zwischen 2 und 5 liegt.

6. Verfahren gemäß einem der Ansprüche 1 bis 5, **dadurch gekennzeichnet, daß** als Reaktoren unter (a) Rührkessel, Schlaufenreaktoren, Strahlschlaufenreaktoren, Strahldüsenreaktoren oder Rohrreaktoren gegebenenfalls mit Umpumpkreislauf verwendet werden.

7. Verfahren gemäß einem der Ansprüche 1 bis 6, **dadurch gekennzeichnet, daß** Anteile des Alkanals in jeden der verwendeten Reaktoren eingebracht werden.

8. Verfahren gemäß einem der Ansprüche 1 bis 7, **dadurch gekennzeichnet, daß** zwischen 20 % und 70 % der insgesamt zugegebenen Menge an Alkanal in den ersten Reaktor eingebracht werden.

9. Verfahren gemäß einem der Ansprüche 1 bis 8, **dadurch gekennzeichnet, daß** das Verfahren so durchgeführt wird, daß der Umsatz des Benzaldehydderivates zwischen 20 % und 95 % beträgt.

10. Verfahren gemäß einem der Ansprüche 1 bis 9, **dadurch gekennzeichnet, daß** wenigstens ein Teil des Alkanals in Mischung mit einem Teil oder der Gesamtmenge des Benzaldehydderivates vorgemischt eingesetzt wird.

11. Verfahren gemäß einem der Ansprüche 1 bis 10, **dadurch gekennzeichnet, daß** das molare Verhältnis aus dem Benzaldehydderivat der Formel II und dem Alkanal der Formel III in der Reaktionsmischung zwischen 5 mol/mol und 100 mol/mol liegt.

12. Verfahren gemäß einem der Ansprüche 1 bis 11, **dadurch gekennzeichnet, daß** der Dihydrozimtaldehyd ausgewählt ist aus der Gruppe Cyclamenaldehyd oder Lysmeral.

13. Verfahren gemäß einem der Ansprüche 1 bis 12, **dadurch gekennzeichnet, daß** die Hydrierung des Zimtaldehydderivats ohne vorherige Reinigung in einem Suspensionsreaktor mit Einbauten durchgeführt wird.

14. Verfahren gemäß einem der Ansprüche 1 bis 13, **dadurch gekennzeichnet, daß** die Katalysatorteilchen der Hydrierung (b) eine mittlere Korngröße von 0,0001 bis 2 mm besitzen.

15. Verfahren gemäß einem der Ansprüche 1 bis 14, **dadurch gekennzeichnet, daß** als Einbauten im Hydrierreaktor unter (b) Gewebe- oder Blechpackungen verwendet werden.

16. Verfahren gemäß einem der Ansprüche 1 bis 15, **dadurch gekennzeichnet, daß** die Querschnittsflächenbelastung von Gas- und Flüssigphase über 50 m³/m²h liegt.

17. Verfahren gemäß einem der Ansprüche 1 bis 16, **dadurch gekennzeichnet, daß** als Reaktor unter (b) ein Strahldüsenreaktor, ein Blasensäulen- oder ein Rohrbündelreaktor verwendet wird.

18. Verfahren gemäß einem der Ansprüche 1 bis 17, **dadurch gekennzeichnet, daß** der Suspensionskatalysator unter (b) als Aktivkomponente mindestens Palladium enthält.

19. Verfahren gemäß Anspruch 18, **dadurch gekennzeichnet, daß** der Anteil an Palladium 0,1 bis 10 Gew.-% beträgt.

20. Verfahren gemäß einem der Ansprüche 18 oder 19, **dadurch gekennzeichnet, daß** der Katalysator auf einem SiO₂, TiO₂, ZrO₂, Al₂O₃ oder Kohlenstoff enthaltenden Träger verwendet wird.

21. Verfahren gemäß einem der Ansprüche 18 bis 20, **dadurch gekennzeichnet, daß** der Katalysator auf einem Aktivkohle enthaltenden Träger verwendet wird.

## Claims

1. A process for the
(a) continuous preparation of cinnamaldehyde derivatives of the formula I 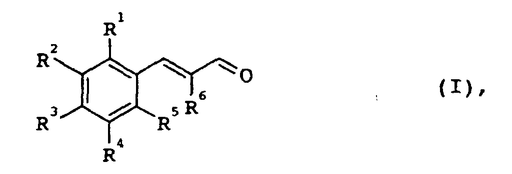 in which R¹, R², R³, R⁴, R⁵ and R⁶, independently of one another, may be hydrogen, C₁- to C₉-alkyl groups or C₁-C₉-alkoxy groups, preferably of cinnamaldehyde derivatives of the formula I in which R¹, R², R³ and R⁶, independently of one another, are hydrogen or C₁-C₉-alkyl groups and R⁴ and R⁵ are hydrogen,
by reaction of benzaldehyde derivatives of the formula II 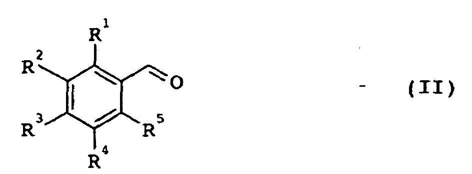 with alkanals of the formula III 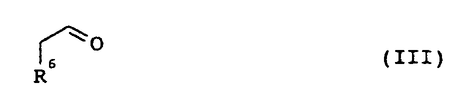 in the presence of bases, which comprises reacting the reactants continuously in a plant consisting of a plurality of reactors in a cascade system, and introducing the alkanal into more than one of the reactors of the plant and
(b) optionally subsequent continuous hydrogenation in a circulation reactor in the presence of a suspension catalyst and hydrogen to give dihydrocinnamaldehyde derivatives of the formula IV, 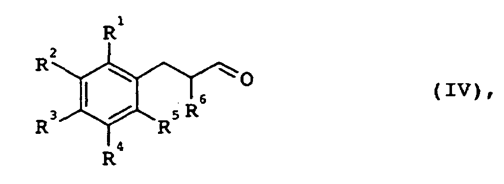
where R¹ to R⁶ have the meanings given above.

2. A process as claimed in claim 1, wherein stage (a) is followed by a hydrogenation as in stage (b).

3. A process as claimed in claim 1 or 2, wherein the benzaldehyde derivative of the formula II used is benzaldehyde itself, m-isopropylbenzaldehyde, p-isopropylbenzaldehyde, p-tert-butylbenzaldehyde, m-tert-butylbenzaldehyde, m-methylbenzaldehyde, p-methylbenzaldehyde, o-methylbenzaldehyde, m-anisaldehyde or p-anisaldehyde, or else mixtures of 2 or more of these benzaldehyde derivatives.

4. A process as claimed in any of claims 1 to 3, wherein the number of reactors used under (a) in the plant is between 2 and 20.

5. A process as claimed in any of claims 1 to 4, wherein the number of reactors used under (a) in the plant is between 2 and 5.

6. A process as claimed in any of claims 1 to 5, wherein the reactors used under (a) are stirred tank reactors, loop reactors, jet loop reactors, jet reactors or tubular reactors, optionally with recirculation circuit.

7. A process as claimed in any of claims 1 to 6, wherein fractions of the alkanal are introduced into each of the reactors used.

8. A process as claimed in any of claims 1 to 7, wherein betweer 20% and 70% of the total amount of alkanal added is introduced into the first reactor.

9. A process as claimed in any of claims 1 to 8, wherein the process is carried out such that the conversion of the benzaldehyde derivative is between 20% and 95%.

10. A process as claimed in any of claims 1 to 9, wherein at least some of the alkanal is used in premixed form in a mixture with some or all of the benzaldehyde derivative.

11. A process as claimed in any of claims 1 to 10, wherein the molar ratio of the benzaldehyde derivative of the formula II and the alkanal of the formula III in the reaction mixture is between 5 mol/mol and 100 mol/mol.

12. A process as claimed in any of claims 1 to 11, wherein the dihydrocinnamaldehyde is chosen from the group consisting of cyclamenaldehyde or lysmeral.

13. A process as claimed in any of claims 1 to 12, wherein the hydrogenation of the cinnamaldehyde derivative is carried out without prior purification in a suspension reactor with internals.

14. A process as claimed in any of claims 1 to 13, wherein the catalyst particles of the hydrogenation (b) have an average particle size of from 0.0001 to 2 mm.

15. A process as claimed in any of claims 1 to 14, wherein the internals used in the hydrogenation reactor under (b) are fabric packings or sheet-metal packings.

16. A process as claimed in any of claims 1 to 15, wherein the superficial velocity of gas phase and liquid phase is more than 50 m³/m²h.

17. A process as claimed in any of claims 1 to 16, wherein the reactor used under (b) is a jet reactor, a bubble-column reactor or a tube-bundle reactor.

18. A process as claimed in any of claims 1 to 17, wherein the suspension catalyst under (b) comprises at least palladium as active component.

19. A process as claimed in claim 18, wherein the proportion of palladium is 0.1 to 10% by weight.

20. A process as claimed in claim 18 or 19, wherein the catalyst is used on a support containing SiO₂, TiO₂, ZrO₂, Al₂O₃ or carbon.

21. A process as claimed in any of claims 18 to 20, wherein the catalyst is used on a support containing activated carbon.

## Revendications

1. Procédé pour
(a) la production en continu de dérivés d'aldéhyde cinnamique de formule générale 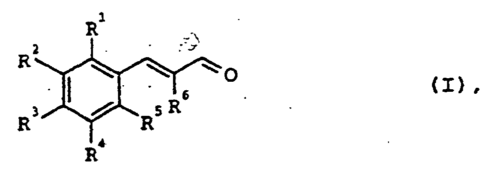 où R¹, R², R³, R⁴, R⁵ et R⁶ peuvent représenter, indépendamment l'un de l'autre, l'hydrogène, des groupes alkyle en C₁ à C₉ ou des groupes alcoxy en C₁-C₉, de préférence de dérivés d'aldéhyde cinnamique de formule I dans laquelle R¹, R², R³ et R⁶ représentent, indépendamment l'un de l'autre, l'hydrogène ou des groupes alkyle en C₁-C₉, tandis que R⁴ et R⁵ désignent l'hydrogène,
par réaction de dérivés de benzaldéhyde de formule générale II 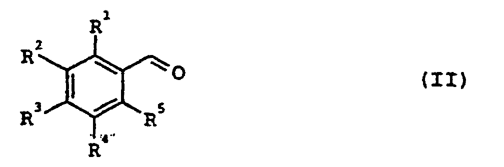 avec des alcanals de formule générale III 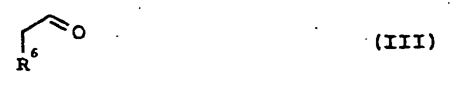 en présence de bases, qui est **caractérisé par le fait que** les composés réagissants sont mis à réagir en continu dans une installation consistant en plusieurs réacteurs reliés en cascade, et que l'alcanal est alors introduit dans plus d'un réacteur de l'installation et
(b) éventuellement l'hydrogénation subséquente en continu dans un réacteur à circulation en présence d'un catalyseur en suspension et d'hydrogène pour former des dérivés d'aldéhyde dihydrocinnamique de formule générale IV, 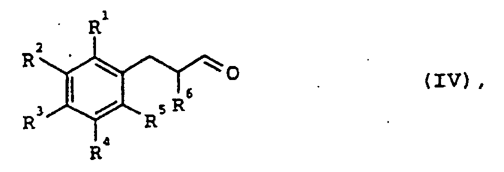
où R¹ à R⁶ ont la signification indiquée plus haut.

2. Procédé selon la revendication 1, **caractérisé par le fait qu'**une hydrogénation selon l'étape (b) a lieu à la suite de l'étape (a).

3. Procédé selon la revendication 1 ou 2, **caractérisé par le fait qu'**on utilise comme dérivé de benzaldéhyde de formule II, le benzaldéhyde lui-même, le m-isopropyl-benzaldéhyde, le p-isopropyl-benzaldéhyde, le p-tert-butyl-benzaldéhyde, le m-tert-butyl-benzaldéhyde, le m-méthyl-benzaldéhyde, le p-méthyl-benzaldéhyde, l'o-méthyl-benzaldéhyde, le m-anisaldéhyde ou le p-anisaldéhyde, ou encore des mélanges de 2 ou plusieurs de ces dérivés de benzaldéhyde.

4. Procédé selon l'une des revendications 1 à 3, **caractérisé par le fait que** le nombre des réacteurs utilisés sous (a) dans l'installation se situe entre 2 et 20.

5. Procédé selon l'une des revendications 1 à 4, **caractérisé par le fait que** le nombre des réacteurs utilisés sous (a) dans l'installation se situe entre 2 et 5.

6. Procédé selon l'une des revendications 1 à 5, **caractérisé par le fait qu'**on utilise comme réacteurs sous (a) des récipients agités, des réacteurs à écoulement en boucles, des réacteurs à boucles en faisceau, des réacteurs à éjecteur à jet ou des réacteurs tubulaires, éventuellement avec recyclage.

7. Procédé selon l'une des revendications 1 à 6, **caractérisé par le fait que** la fraction alcanal est introduite dans chacun des réacteurs utilisés.

8. Procédé selon l'une des revendications 1 à 7, **caractérisé par le fait qu'**entre 20 % et 70 % de la quantité d'alcanal ajoutée au total sont introduits dans le premier réacteur.

9. Procédé selon l'une des revendications 1 à 8, **caractérisé par le fait que** le procédé est mis en oeuvre de telle manière que la conversion du dérivé de benzaldéhyde se situe entre 20 % et 95 %.

10. Procédé selon l'une des revendications 1 à 9, **caractérisé par le fait qu'**au moins une partie de l'alcanal est introduite prémélangée avec une partie ou la quantité totale du dérivé de benzaldéhyde.

11. Procédé selon l'une des revendications 1 à 10, **caractérisé par le fait que** le rapport molaire entre le dérivé de benzaldéhyde de formule II et l'alcanal de formule III dans le mélange réactionnel se situe entre 5 mol/mol et 100 mol/mol.

12. Procédé selon l'une des revendications 1 à 11, **caractérisé par le fait que** l'aldéhyde dihydrocinnamique est choisi dans le groupe de l'aldéhyde de cyclamen ou du lysméral.

13. Procédé selon l'une des revendications 1 à 12, **caractérisé par le fait que** l'hydrogénation du dérivé d'aldéhyde cinnamique est effectuée sans purification préalable dans un réacteur pour suspension avec éléments incorporés.

14. Procédé selon l'une des revendications 1 à 13, **caractérisé par le fait que** les particules de catalyseur de l'hydrogénation (b) possèdent un calibre moyen de 0,0001 à 2 mm.

15. Procédé selon l'une des revendications 1 à 14, **caractérisé par le fait qu'**on utilise comme éléments incorporés dans le réacteur d'hydrogénation sous (b) des garnissages de tissu ou de tôle.

16. Procédé selon l'une des revendications 1 à 15, **caractérisé par le fait que** la charge par unité de section de la phase gazeuse ou fluide se situe au-dessus de 50 m³/m².h.

17. Procédé selon l'une des revendications 1 à 16, **caractérisé par le fait qu'**on utilise comme réacteur sous (b) un réacteur à éjecteur à jet, un réacteur à colonnes à bulles ou un réacteur à faisceau tubulaire.

18. Procédé selon l'une des revendications 1 à 17, **caractérisé par le fait que** le catalyseur en suspension sous (b) contient comme composant actif au moins du palladium.

19. Procédé selon la revendication 18, **caractérisé par le fait que** la proportion de palladium est de 0,1 à 10 % en poids.

20. Procédé selon l'une des revendications 18 ou 19, **caractérisé par le fait que** le catalyseur est utilisé sur un support contenant SiO₂, TiO₂, ZrO₂, Al₂O₃ ou du carbone.

21. Procédé selon l'une des revendications 18 à 20, **caractérisé par le fait que** le catalyseur est utilisé sur un support contenant du charbon actif.
